# EUROPEAN PATENT SPECIFICATION

(11) **EP 1 216 042 B1**
(45) Date of publication and mention of the grant of the patent: **13.12.2006**
(21) Application number: 00965255.3
(22) Date of filing: 21.09.2000
(51) Int. Cl.: A61K 31/337, A61P 35/00, A61K 9/51

(54) **FORMULATIONS OF PACLITAXEL ENTRAPPED INTO NANOPARTICLES OF POLYMERIC MICELLES**
ARZNEIZUBEREITUNGEN ENTHALTEND PACLITAXEL EINGESCHLOSSEN IN NANOPARTIKELN VON POLYMERISCHEN MIZELLEN
FORMULATIONS DE PACLITAXEL CONTENUS DANS DES NANOPARTICULES DE MICELLES POLYMERIQUES

(30) Priority: 23.09.1999 US 401927; 11.07.2000 IN DE064100
(43) Date of publication of application: 26.06.2002
(73) Proprietor: DABUR PHARMA LTD., Safdarjung Hospital, New Delhi 110 029 (IN)
(72) Inventor: BURMAN, Anand, C., Ghaziabad 201 010 Uttar Pradesh (IN); MUKHERJEE, Rama, Ghaziabad 201 010 Uttar Pradesh (IN); KHATTAR, Dhiraj, Ghaziabad 201 010 Uttar Pradesh (IN); KUMAR, Mukesh, Ghaziabad 201 010 Uttar Pradesh (IN); BALA, Honey, Ghaziabad 201 010 Uttar Pradesh (IN); SHRIVASTAVA, Rajiv Kumar, Ghaziabad 201 010 Uttar Pradesh (IN)
(74) Representative: Ebner von Eschenbach, Jennifer
(86) International application number: PCT/US2000/025914
(87) International publication number: WO 2001/021174

(56) References cited:
- EP-A- 0 583 955
- WO-A-97/10849
- DATABASE WPI Section Ch, Week 200008 Derwent Publications Ltd., London, GB; Class A25, AN 2000-092595 XP002161131 & JP 11 335267 A (NANOCARRIER KK), 7 December 1999 (1999-12-07)

## Description

This invention relates to pharmaceutical formulations of paclitaxel, or its derivatives entrapped into nanoparticles of co-polymeric micelles, a process for preparing the same and the use thereof.

### BACKGROUND OF THE INVENTION

Amongst the chemotherapeutic agents that have entered clinical testing in the last decade paclitaxel is one of the most promising candidates. It has shown impressive activities against ovarian cancer, breast cancer, non-small cell lung cancer, small cell lung cancer, squamous cell cancer of the head and neck and malignant melanomas. It is also undergoing clinical trials against several other malignancies.

It is preferred that paclitaxel be administered parenterally. Unfortunately, paclitaxel and many of its derivatives and analogs have exceedingly low solubilities in most physiologically acceptable aqueous solvents that would be compatible with intravascular administration.

There is one approved formulation of paclitaxel for parenteral administration in humans. This formulation contains the drug, 527 mg/ml of polyoxyethylated castor oil (Cremophor EL) and 49.7% v/v of absolute ethanol. Unfortunately, Cremophor has a potential to cause hypersensitivity reactions. The most common side effects of the available paclitaxel formulation are severe: hypotension, urticaria, angioedema and most notably anaphylactoid reactions with a risk of a fatal outcome. These serious side effects from the current drug formulation have made it necessary to pre-medicate the patients with diphendydramine, histamine H₂ antagonists or even corticosteroids.

Therefore, there is need for alternate formulations of paclitaxel, its derivatives or analogs. The present invention provides composition that makes it possible to reduce the ethanol concentration greatly, and to eliminate Cremophor completely from the formulations.

The formulations disclosed herein, contain nanoparticles of polymeric micelles that entrap/solubilize taxane analogs like paclitaxel without affecting their cytotoxic properties.

Nanometer size drug carriers with hydrophilic surfaces are found to evade recognition and uptake by the reticulo-endothelial systems (RES) and thus can circulate in the blood for a long time. Another advantage of these hydrophilic nanoparticles is that, due to their extremely small size, the particles extravasate at the pathological sites such as solid tumors through passive targeting mechanism.

These nanoparticles of polymeric micelles besides keeping the drug in aqueous solution also help in increasing the circulation time in blood, in vivo.

### SUMMARY OF THE INVENTION

The object if this invention is to overcome the drawbacks in the prior art by providing alternate formulations of paclitaxel, or its derivatives by entrapping the drug in nanoparticles of polymeric micelles.

An important object of this invention is a process for the preparation of formulations of nanoparticles of polymeric micelles loaded with paclitaxel, or its derivatives dispersed in aqueous solution, which can be diluted with aqueous intravenous fluids.

A further object of this invention is the use of formulations of this invention for the treatment of conditions arising out of excessive proliferation of cells.

Another object of this invention is the use of the formulations of this invention to target maximum amounts of drug to tumors and only negligible amounts to other tissues, which obviates the disadvantages associated with the prior art.

The formulations of this invention contain nanoparticles of polymeric micelles which contain paclitaxel or a derivative thereof physically entrapped therein; said formulation further comprising an alcohol, a co-polymer, an anionic surfactant, a buffering agent and an intravenous aqueous diluting fluid.

### BRIEF DESCRIPTION OF THE FIGURE

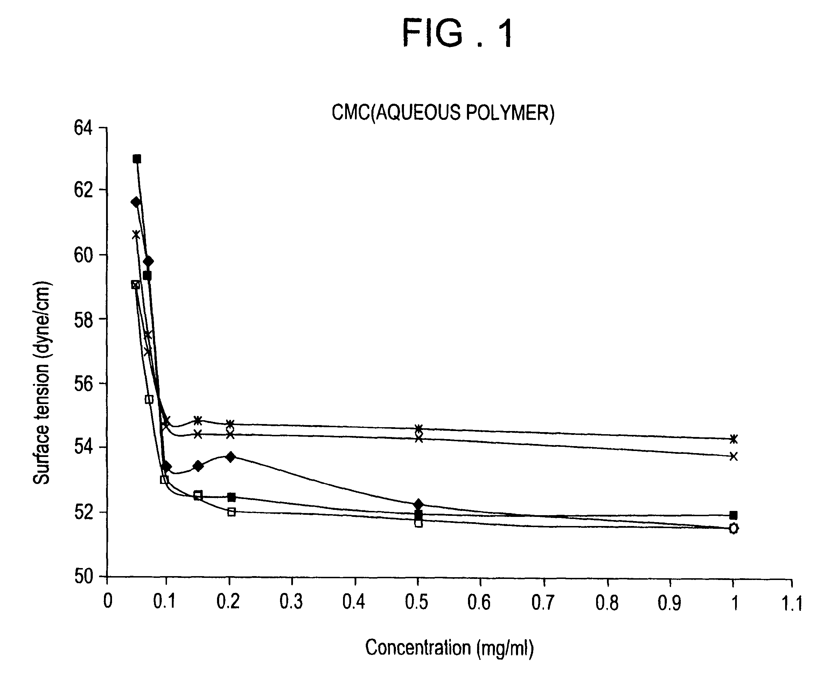

Figure 1 shows the critical micelle concentration (CMC) of the polymer.

### DETAILED DESCRIPTION OF THE INVENTION

The present invention provides compositions and methods for the solubilization of taxane analogs (e.g. paclitaxel) in nanoparticles of polymeric micelles in a pharmaceutically acceptable liquid vehicle that avoids the use of polyoxylethylated castor oils such as Cremophor EL, such that the drugs remain physically and chemically stable and can be administered intravascularly without undue toxicity from the undissolved drug and/or from the vehicle at drug doses contemplated to be effective to exhibit clinically significant effects on the excessive proliferation of cells (e.g. having significant anti-tumor activity). In a broader sense, the present invention describes a method to administer poorly water-soluble taxane analogs such as paclitaxel intravascularly. This circumvents the poor intestinal absorption of the drug as well as avoids the serious systemic adverse effects of Cremophor.

A detailed method of synthesis of the co-polymer for preparing the nanoparticles is described in U.S. Patent Application No. 09/401,927 filed in the U.S. PTO on September 23, 1999. Nanoparticles are made of the co-polymer described in U.S. Patent Application 09/401,927. The polymer is first dissolved in a diluting fluid (e.g. dextrose solution) and anionic surfactant (e.g. sodium deoxycholate). The pH is adjusted to between 6.0 and 7.5 with a buffering agent. The drug (e.g. paclitaxel) is then loaded into these void nanoparticles. For this purpose the drug is dissolved in a suitable amount of an alcohol (e.g, preferably ethanol) and this solution of drug is then added to the solution of void nanoparticles. Immediately on addition, the drug moves from the hydrophilic aqueous environment to the hydrophobic core of the nanoparticles which are further stabilized by the anionic surfactant present on the surface of the nanoparticles.

The formulations of this invention can be administered parenterally or intravenously with a clinically acceptable, aqueous dilution fluid.

The compositions are prepared as described in U.S. Patent Application 09/401,927 and as described below:
a) dissolving at least one type of amphiphilic monomer, preferably two types of amphiphilic monomers in an aqueous medium to obtain micelles;
b) adding an aqueous cross-linking agent and optionally an activator and initiator;
c) subjecting the mixture to polymerization in the presence of an inert gas at 20°C to 80°C, preferably between 30°C to 40°C, until the polymerization of the micelles is complete;
d) purifying the nanoparticles of the co-polymeric micelles by dialysis to remove toxic monomers and other unreacted materials;
e) after purification as above, the solution of the void nanoparticles is sterilized by filtration and stored as such or optionally, lyophilized and stored for use later after dissolving in an intravenous dilution fluid;
f) adding an anionic surfactant;
g) adjusting the pH with a suitable buffering agent;
h) dissolving paclitaxel, or its derivatives, in a suitable solvent, generally an alcohol preferably ethanol and adding this solution to the nanoparticle solution as a rapid fine stream;
i) optionally lypholizing the nanoparticles of co-polymerized micelles containing entrapped paclitaxel, or its derivatives to obtain dry powder, and optionally;
j) reconstituting the nanoparticles in suitable medium for human or mammalian administration.

The completion of polymerization of the monomers in step c) is determined by monitoring the depletion of the monomers from the reaction mixture by HPLC.

### Dialysis is carried out for 2-4 hours to eliminate unreacted monomers.

Nanoparticles of co-polymeric micelles are formed by the reaction/polymerization of the monomers in the reaction mixture. Random polymer chains are formed and are then cross-linked with each other with the help of a cross-linking agent. The amount of the cross-linking agent affects the amount of cross-linking in the polymer which in turn affects the compactness of the network formed. The compactness of this network has a direct bearing on the drug entrapment and consequently drug release from these nanoparticles. The more compact the network, the more difficult it is for the drug to be released.

The hydrophobic cores of these nanoparticles of co-polymeric micelles are composed of hydrophobic part of the co-polymers with the hydrophilic part extended outside towards the aqueous medium.

Amphiphilic monomers which form polymers through radical polymerization reaction are preferred. Preferred monomers are vinyl pyrrolidone, acrylic acid, alkyl acrylates having a chain length of C₃-C₆ and/or functionalized polyethylene glycol of a molecular weight 2000 to 6000, N-alkylacrylamide having a chain length of C₃ to C₆ and alkylcyanoacrylate having a chain length of C₃ to C₆. Two or more amphiphilic monomers are used.

A functionalized polyethylene glycol is a polyethylene glycol reacted to another organic compound containing a functional group. A preferred functionalized polyethylene glycol is polyethylene glycol ester of maleic anhydride. Polyethyleneglycol is reacted with maleic anhydride to form polyethylene glycol ester of maleic anhydride. Functionalized polyethyleneglycol may be covalently attached to the polymer chain of the nanoparticles of polymeric micelles with the polyethylene moiety protruding outside on the surface of the nanoparticles.

A preferred combination of amphiphilic monomers is vinylpyrrolidone and N-isopropyl acrylamide in the molar ratio of 10-50:50-90. Another preferred combination of amphilic monomers in vinylpyrrolidone, N-isopropyl acrylamide and monoester of polyethylene glycol maleic anhydride.

The buffering agents must be suitable for intravenous products. The buffering agent may be selected from acetate, borate, citrate, phosphate, or phthlate buffers, or agents such as diethanolamine, glycine, or glutamic acid.

The cross-linking agent whenever used is at least a bi-functional vinyl derivative. It can be more than bi-functional (i.e. it can have more than two reactive sites). A bi-functional vinyl derivative that can be used is N,N'-methylene bis acrylamide.

The initiators may be peroxide compounds, such as diacyl peroxide compounds such as benzoyl peroxide, diacetyl peroxide or dialkyl peroxides such as tertiary butyl peroxide and tertiary amyl peroxide or perdisulphate of 2,2'-azo bis isobutyronitrile.

Activators may be selected from tetramethylethylene diamine (TMED) and ferrous ammonium sulphate.

Any combination of initiator and activator can be used. Two or more initiators can be used. Two or more activators can be used.

The inert gas may be a gas such as nitrogen or argon.

The preferred anionic surfactant is sodium deoxycholate.

The dilution fluid may be selected from but is not limited to water, saline, dextrose 5% solution, dextrose 10% solution, dextrose and sodium chloride solution, sodium lactate solution, lactated Ringer solution, mannitol solution, mannitol with dextrose or sodium chloride solution, Ringer's solution, sodium chloride solution, sterile water for injection and multiple electrolyte solutions comprising varying combinations of electrolytes, dextrose, fructose and invert sugar. Preferably the dilution fluid is a fluid comprising dextrose and water.

Derivatives include but are not limited to;
1. 2-debenzoyl-2-methazidobenzoylpaclitaxel
2. Taxotere
3. Ring A contracted paclitaxel Derivatives
4. 10-Deacetyl Paclitaxel
5. 7-Deoxy Paclitaxel
6. Oxetane Ring (ring D) modified Paclitaxel
7. 2-Deoxy Paclitaxel
8. 2-Aroyl-2-Debenzoyl paclitaxel analogues
9. N-Benzoyl Modified Paclitaxel analogues
10. 2,3 cyclohexyl Paclitaxel analogues
11. 4-Deacetyl Paclitaxel analogues
12. 7,8-cyclopropane Paclitaxel
13. 7-Fluoropaclitaxel

The % loading of paclitaxel, or its derivatives with respect to the polymer means that if in the formulation the concentration of the polymer is, for example, 1 mg/ml and the concentration of paclitaxel is 0.2 mg/ml, the drug is 20% by weight of the polymer. Since the total amount of the drug goes inside or is loaded in the nanoparticles, the drug loading is 20% by weight with respect to the polymer. The drug can be added up to a maximum loading of 400% w/w in the nanoparticles of co-polymeric micelles.

The concentration of the paclitaxel, or derivatives in the alcohol is 10 to 80 mg/ml.

The concentration of the co-polymer is from about 0.01 to 20 mg/ml, preferably 0.1 to 1mg/ml of the formulation.

The preferred concentration of the anionic surfactant is 0.01 to 0,5 mg/ml of the formulation.

The formulations described herein may be utilized to dissolve paclitaxel, its analogues or its derivatives in concentrations ranging from 0.1 to more than 18 mg/ml of the formulation. This range is contemplated to cover the administration of dosages necessary to yield active cytotoxic concentrations, in vivo to treat the conditions such as malignancies sensitive to these drugs.

The stable and parenterally acceptable novel formulations of nanoparticles of paclitaxel, its derivatives or its analogs can be utilized for the treatment of pathological conditions arising out of excessive proliferation of cells such as rheumatoid arthritis or cancer. The formulations can be used to treat, cancers such as ovarian cancer, breast cancer, non-small cell lung cancer, small cell lung cancer, squamous cell cancer of the head and neck and malignant melanomas.

The formulations of this invention retain full cytotoxic activity as assessed in xenographs of malignant cells in mice. Exemplary formulations as shown herein have been found to be effective in causing regression of tumors of oral squamous cell carcinoma and murine metastatic melanomas in mice xenographs.

The following non-limiting examples are included to demonstrate preferred embodiments of the invention.

### EXAMPLES 1 - 9

The polymer (5mg) was dissolved in 5 ml of the diluting fluid followed by the addition of the anionic surfactant sodium deoxycholate (5mg) to obtain a clear solution. Paclitaxel solution in absolute alcohol (20 mg/ml) was then added to the solution of polymer and the surfactant to obtain drug concentrations of 0.1, 0.15 and 0.2 mg/ml. Different diluting fluids were tried and the stability of the resulting drug solutions are tabulated below:

| Dilution Fluid | Polymer Conc. (mg/ml | Sodium DOC Conc (mg/ml) | Paclitaxel Conc. (mg/ml) | % loading of Paclitaxel w.r.t. Polymer | Stability |
|---|---|---|---|---|---|
| Water for Injection | 1 | 1 | 0.10 | 10% | < 1hrs |
| Water for Injection | 1 | 1 | 0.1 5 | 15% | < 1hrs |
| Water for Injection | 1 | 1 | 0.20 | 20% | < 1hrs |
| Normal Saline | 1 | 1 | 0.10 | 10% | < 1hrs |
| Normal Saline | 1 | 1 | 0.15 | 15% | < 1hrs |
| Normal Saline | 1 | 1 | 0.20 | 20% | < 1hrs |
| 5 % Dextrose | 1 | 1 | 0.10 | 10% | > 8hrs |
| 5 % Dextrose | 1 | 1 | 0.15 | 15% | > 6hrs |
| 5 % Dextrose | 1 | 1 | 0.20 | 20% | > 4hrs |

Dextrose had a stabilizing effect on the drug solubility as reflected in the stability of the drug solution.

### EXAMPLES 10 - 12

### EFFECT OF HIGHER DRUG LOADING

The polymer (5mg) was dissolved in 5 ml of the diluting fluid followed by the addition of the anionic surfactant sodium deoxycholate (5mg) to obtain a clear solution. Paclitaxel solution in absolute alcohol (20 mg/ml) was then added to the solution of polymer and the surfactant to get drug concentrations of 0.2, 0.25 and 0.3 mg/ml. The stability of the resulting drug solutions are tabulated below:

| Dilution Fluid | Polymer Conc. (mg/ml | Sodium DOC Conc (mg/ml) | Paclitaxel Conc. (mg/ml) | % loading of Paclitaxel w.r.t. Polymer | Stability |
|---|---|---|---|---|---|
| 5 % Dextrose | 1 | 1 | 0.20 | 20% | > 4hrs |
| 5 % Dextrose | 1 | 1 | 0.25 | 25% | < 4hrs |
| 5 % Dextrose | 1 | 1 | 0.30 | 30% | < 4hrs |

More than 20% drug loading with respect to the polymer resulted in reduced stability of the drug solution.

### EXAMPLES 13 - 14

### EFFECT OF HIGHER CONCENTRATION OF DEXTROSE

The polymer (5mg) was dissolved in 5 ml of the diluting fluid followed by the addition of the anionic surfactant sodium deoxycholate (5mg) to obtain a clear solution. Paclitaxel solution in absolute alcohol (20 mg/ml) was then added to the solution of polymer and the surfactant to obtain drug concentrations of 0.2 mg/ml. Different diluting fluids were tried and the stability of the resulting drug solutions are tabulated below:

| Dilution Fluid | Polymer Conc. (mg/ml | Sodium DOC Conc (mg/ml) | Paclitaxel Conc. (mg/ml) | % loading of Paclitaxel w.r.t. Polymer | Stability |
|---|---|---|---|---|---|
| 5 % Dextrose (pH 5.1) | 1 | 1 | 0.20 | 20% | > 4hrs |
| 10 % Dextrose (pH 5.1) | 1 | 1 | 0.20 | 20% | > 6hrs |

10% dextrose had a more stabilizing effect on the solution as compared to 5% dextrose. The same was also reflected on the better solution clarity with 10% dextrose.

### EXAMPLES 15-17

### EFFECT OF PH ON SOLUTION STABILITY AT 20% DRUG LOADING

The polymer (5mg) was dissolved in 5 ml of the diluting fluid followed by the addition of the anionic surfactant sodium deoxycholate (5mg) to obtain a clear solution. pH of the resulting solution was adjusted with sodium citrate. Paclitaxel solution in absolute alcohol (20 mg/ml) was then added to the solution of polymer and the surfactant to obtain drug concentrations of 0.2 mg/ml. Results are tabulated below:

| Dilution Fluid | Polymer Conc. (mg/ml | Sodium DOC Conc (mg/ml) | Sodium Citrate. (mg/ml) | pH | Paclitaxel Conc. (mg/ml) | Stability |
|---|---|---|---|---|---|---|
| 10 % Dextrose (pH 3.9) | 1 | 1 | 0.00 | 6.28 | 0.20 | < 1hrs |
| 10 % Dextrose (ph 3.9) | 1 | 1 | 0.06 | 6.36 | 0.20 | > 6hrs |
| 10 % Dextrose (ph 3.9) | 1 | 1 | 0.12 | 6.53 | 0.20 | > 4hrs |

### EXAMPLE 18

### DETERMINATION OF CMC OF POLYMER

Critical Micelle Concentration (CMC) of the polymer was determined using aqueous solutions of concentration ranging from 0.01 to 1.0 mg/ml. CMC was found to be in the range of 0.1 - 0.2 mg/ml as shown in Figure 1.

### EXAMPLES 19 - 23

### EFFECT OF REDUCTION OF POLYMER/SODIUM DOC ON DRUG LOADING

The requisite amount of polymer was dissolved in 5 ml of the diluting fluid followed by the addition of requisite amount of anionic surfactant sodium deoxycholate to obtain the concentrations shown in the table below. pH of the resulting solution was adjusted to 6.4 - 6.8 with sodium citrate. Paclitaxel solution in absolute alcohol (20 mg/ml) was then added to the solution of polymer and the surfactant to obtain drug concentrations of 0.2 mg/ml. Results are tabulated below:

| Dilution Fluid | Polymer Conc. (mg/ml) | Sodium DOC. (mg/ml) | pH | Paclitaxel Conc. (mg/ml) | Stability |
|---|---|---|---|---|---|
| 10% Dextrose | 1 | 1 | 6.45 | 0.20 | > 6 hrs |
| 10% Dextrose | 0.5 | 0.5 | 6.44 | 0.20 | > 6 hrs |
| 10% Dextrose | 0.25 | 0.25 | 6.46 | 0.20 | > 6 hrs |
| 10% Dextrose | 0.125 | 0.125 | 6.47 | 0.20 | > 6 hrs |
| 10% Dextrose | 0.0625 | 0.0625 | 6.44 | 0.20 | < 6 hrs |

### EXAMPLES 24 - 27

### EFFECT OF REDUCTION OF SODIUM DOC ON DRUG LOADING

The polymer (0.625 mg) was dissolved in 5 ml of the diluting fluid followed by the addition of the anionic surfactant sodium deoxycholate to obtain a clear solution. pH of the resulting solution was adjusted to 6.4 - 6.8 with sodium citrate.

Paclitaxel solution in absolute alcohol (20 mg/ml) was then added to the solution of polymer and the surfactant to obtain drug concentrations of 0.2 mg/ml. Results are tabulated below:

| Dilution Fluid | Polymer Conc. (mg/ml) | Sodium DOC. (mg/ml) | Paclitaxel Conc. (mg/ml) | Stability |
|---|---|---|---|---|
| 10 % Dextrose | 0.125 | 0.03125 | 0.20 | < 1 hrs |
| 10 % Dextrose | 0.125 | 0.0625 | 0.20 | < 2 hrs |
| 10 % Dextrose | 0.125 | 0.09375 | 0.20 | > 6 hrs |
| 10% Dextrose | 0.125 | 0.125 | 0.20 | > 6 hrs |

### EXAMPLES 28 - 35

### OPTIMIZATION OF DRUG LOADING

The requisite amount of the polymer was dissolved in 5 ml of the diluting fluid followed by the addition of requisite amount of the anionic surfactant sodium deoxycholate to obtain the concentrations shown in the table below. pH of the resulting solution was adjusted to 6.4 - 6.8 with sodium citrate. Paclitaxel solution in absolute alcohol (20 mg/ml) was then added to the solution of polymer and the surfactant to get drug concentrations of 0.2, 0.5, 0.6, 0.7, 0.8, 2.4, 3.2, 4.0, and 10 mg/ml. Results are tabulated below:

| Dilution Fluid | Polymer Conc. (mg/ml) | Sodium DOC. (mg/ml) | Paclitaxel Conc. (mg/ml) | Stability |
|---|---|---|---|---|
| 10% Dextrose | 0.125 | 0.125 | 0.20 | > 6 hrs |
| 10 % Dextrose | 0.125 | 0.125 | 0.50 | < 4 hrs |
| 10% Dextrose | 0.125 | 0.125 | 0.80 | < 2 hrs |
| 10 % Dextrose | 0.125 | 0.125 | 2.4 | < 1 hrs |
| 10 % Dextrose | 0.125 | 0.125 | 3.2 | < 30 min |
| 10 % Dextrose | 0.125 | 0.125 | 4.0 | < 15 min |
| 10 % Dextrose | 0.125 | 0.125 | 10.0 | < 5 min |
| 10 % Dextrose | 0.2 | 0.2 | 0.80 | < 3hrs |
| 10 % Dextrose | 0.3 | 0.3 | 0.80 | < 2 hrs |
| 10 % Dextrose | 0.3 | 0.3 | 0.70 | < 4 hrs |
| 10% Dextrose | 0.3 | 0.3 | 0.60 | < 6 hrs |
| 10% Dextrose | 0.3 | 0.3 | 0.50 | > 6 hrs |

### EXAMPLES 36 - 40

### EFFECT OF REDUCTION OF SODIUM DOC ON DRUG LOADING

The polymer (1.5 mg) was dissolved in 5 ml of the diluting fluid followed by the addition of the anionic surfactant sodium to obtain concentrations of 0, 0.10, 0.15, 0.20 and 0.25 mg/ml. pH of the resulting solution was adjusted to 6.4 - 6.8 with sodium citrate. Paclitaxel solution in absolute alcohol (20 mg/ml) was then added to the solution of polymer and the surfactant to obtain drug concentrations of 0.6 mg/ml. Results are tabulated below:

| Dilution Fluid | Polymer Conc. (mg/ml) | Sodium DOC. (mg/ml) | Paclitaxel Conc. (mg/ml) | Stability |
|---|---|---|---|---|
| 10 % Dextrose | 0.3 | 0 | 0.60 | < 30 min |
| 10 % Dextrose | 0.3 | 0.10 | 0.60 | < 1 hrs |
| 10 % Dextrose | 0.3 | 0.15 | 0.60 | < 4 hrs |
| 10% Dextrose | 0.3 | 0.2 | 0.60 | >8hrs |
| 10% Dextrose | 0.3 | 0.25 | 0.60 | > 8 hrs |

### THE PREFERRED COMPOSITION

Drug Solution is prepared by dissolving paclitaxel (100 mg) in ethanol (5 ml) to obtain a clear solution having a concentration of 20 mg/ml and filtered through 0.2µ filter.

Infusion Vehicle is obtained by separately dissolving 30 mg of the polymer, 30 mg of sodium citrate and 20 mg of the anionic surfactant sodium deoxycholate in 100 ml of Dextrose (10%) solution and filtering through 0.2µ filter.

### FORMULATION FOR INFUSION

The required amount of the drug solution (3 ml) is added to the vehicle to obtain a drug concentration of 0.6 mg/ml. The perfusion fluid is stable for more than 12 hrs without any apparent signs of precipitation of drug. Stability is also indicated by the fact that more than 90% of the drug is available in solution form at the end of 24 hrs, when analyzed by HPLC.

### LOSS OF DRUG DURING PASSAGE THROUGH 0.2µ IN-LINE FILTER IN A I.V. SET

A formulation of the preferred composition (100 ml) as given above was passed through an infusion set containing 0.2µ in-line filter, at a flow rate of about 3 ml per minute to simulate the actual bedside situation. The loss of drug during passage through the 0.2µ in-line filter was not more than 5%.

## Claims

1. A formulation containing nanoparticles of polymeric micelles containing a drug selected from paclitaxel, or a derivative thereof physically entrapped therein; said formulation further comprising an alcohol, a co-polymor, an anionic surfactant, a buffering agent and an intravenous aqueous diluting fluid.

2. The formulation as claimed in claim 1, wherein the copolymer is formed from at least two amphiphilic monomers selected from the group consisting of vinylpyrrolidone, acrylic acid, alkyl acrylates having a chain length of C₃ -C₆, functionalized polyethylene glycol of a molecular weight of 2000 to 6000, N-alkylacrylamide having a chain length of C₃ to C₆ and alkylcyanoacrylate having a chain length of C₃ to C₆.

3. The formulation as claimed in claim 1 or 2 wherein the co-polymer is formed from vinylpyrrolidone, N-isopropyl acrylamide, and functionalized polyethylene glycol.

4. The formulation as claimed in claim 1, 2 or 3 wherein the concentration of the drug about 0.1 to 20 mg/ml of the formulation.

5. The formulation as claimed in claim 1, 2, 3 or 4 wherein the concentration of copolymer is about 0.01 to 20 mg/ml of the formulation.

6. The formulation as claimed in anyone of claims 1 to 5 wherein the anionic surfactant is sodium deoxycholate.

7. The formulation as claimed in claim 6, wherein the concentration of sodium deoxycholate is about 0.01 to 20 mg/ml of the formulation.

8. The formulation as claimed in claim 1, wherein the buffering agent is suitable for intravenous administration.

9. The formulation a claimed in anyone of claims 1 to 6 wherein the pH is between 6.0 to 7.5.

10. The formulation as claimed in claim 1, wherein the intravenous aqueous diluting fluid is a dextrose solution.

11. The formulation as claimed in any one of claims 1 to 6 wherein the concentration of paclitaxel in the alcohol is 10 to 80 mg/ml.

12. A method of preparing a formulation according to claim 1, 2, 3, 4, 5, 6 or 11 for intravascular administration comprising the steps of:
a) dissolving a co-polymer in an intravenous dilution fluid;
b) adding an anionic surfactant to the solution of step "a";
c) adjusting the pH of the resulting solution; and
d) adding an alcoholic solution of paclitaxel or a derivative thereof to the above solution.

13. A formulation prepared by the method of claim 12.

14. A formulation as claimed in claim 1, 2, 3, 4, 5, 6 or 11 for treating a condition arising from excessive proliferation of cells.

15. A formulation according to claim 14, wherein the condition is cancer.

16. A formulation according to claim 14, wherein the condition is a tumor.

17. A formulation according to claim 14, wherein the condition is rheumatoid arthritis.

18. Use of a formulation as claimed in claim 1, 2, 3, 4, 5*,* 6 or 11 for the manufacture of a medicament for treating a condition arising from the excessive proliferation of cells.

## Patentansprüche

1. Zubereitung, enthaltend Nanopartikel von polymeren Micellen, die ein Arzneimittel enthalten, das ausgewählt ist aus Paclitaxel oder einem Derivat davon, das mechanisch darin eingeschlossen ist, wobei die Zubereitung ferner einen Alkohol aufweist, ein Copolymer, ein Aniontensid, ein Puffermittel und ein intravenöses, wässriges Verdünnungsfluid.

2. Zubereitung nach Anspruch 1, wobei das Copolymer erzeugt wird aus mindestens zwei amphiphilen Monomeren, die ausgewählt sind aus der Gruppe, bestehend aus Vinylpyrrolidon, Acrylsäure, Alkylacrylaten mit einer Kettenlänge von C₃ bis C₆, funktionalisiertem Polyethylenglykol einer relativen Molekülmasse von 2.000 bis 6.000, N-Alkylacrylamid mit einer Kettenlänge von C3 bis C6 und Alkylcyanoacrylat mit einer Kettenlänge von C3 bis C6.

3. Zubereitung nach Anspruch 1 oder 2, wobei das Copolymer erzeugt wird aus Vinylpyrrolidon, N-Isopropylacrylamid und funktionalisiertem Polyethylenglykol.

4. Zubereitung nach Anspruch 1, 2 oder 3, worin die Konzentration des Arzneimittels etwa 0,1 bis 20 mg/ml der Zubereitung beträgt.

5. Zubereitung nach Anspruch 1, 2, 3 oder 4, worin die Konzentration des Copolymers etwa 0,01 bis 20 mg/ml der Zubereitung beträgt.

6. Zubereitung nach einem der Ansprüche 1 bis 5, worin das Aniontensid Natriumdesoxycholat ist.

7. Zubereitung nach Anspruch 6, worin die Konzentration von Natriumdesoxycholat etwa 0,01 bis 20 mg/ml der Zubereitung beträgt.

8. Zubereitung nach Anspruch 1, wobei das Puffermittel zur intravenösen Verabreichung geeignet ist.

9. Zubereitung nach einem der Ansprüche 1 bis 6, worin der pH-Wert zwischen 6,0 und 7,5 liegt.

10. Zubereitung nach Anspruch 1, worin das intravenöse, wässrige Streckfluid eine Dextroselösung ist.

11. Zubereitung nach einem der Ansprüche 1 bis 6, worin die Konzentration von Paclitaxel im Alkohol 10 bis 80 mg/ml beträgt.

12. Verfahren zum Herstellen einer Zubereitung nach Anspruch 1, 2, 3, 4, 5, 6 oder 11 zur intravaskulären Verabreichung, umfassend die Schritte:
a) Auflösen eines Copolymers in einem intravenösen Verdünnungsfluid;
b) Zugeben eines Aniontensids zu der Lösung von Schritt "a";
c) Einstellen des pH-Werts der resultierenden Lösung; und
d) Zugeben der alkoholischen Lösung von Paclitaxel oder einem Derivat davon zu der vorgenannten Lösung.

13. Zubereitung, hergestellt nach dem Verfahren von Anspruch 12.

14. Zubereitung nach Anspruch 1, 2, 3, 4, 5, 6 oder 11 zur Behandlung einer Erkrankung, die aus einer übermäßigen Proliferation von Zellen entsteht.

15. Zubereitung nach Anspruch 14, wobei die Erkrankung Krebs ist.

16. Zubereitung nach Anspruch 14, wobei die Erkrankung ein Tumor ist.

17. Zubereitung nach Anspruch 14, wobei die Erkrankung rheumatoide Arthritis ist.

18. Verwendung einer Zubereitung nach Anspruch 1, 2, 3, 4, 5, 6 oder 11, für die Herstellung eines Medikaments zur Behandlung einer Erkrankung, die aus der übermässigen Proliferation von Zellen entsteht.

## Revendications

1. Formulation contenant des nanoparticules de micelles polymères contenant un médicament choisi parmi le paclitaxel, ou un dérivé de celui-ci, physiquement inclus en son sein ; ladite formulation comprenant en outre un alcool, un copolymère, un agent tensioactif anionique, un agent tampon et un fluide de dilution aqueux intraveineux.

2. Formulation selon la revendication 1, dans laquelle le copolymère est formé à partir d'au moins deux monomères amphiphiles choisis dans le groupe comprenant la vinylpyrrolidone, l'acide acrylique, les acrylates d'alkyle ayant une longueur de chaîne en C₃ à C₆, un polyéthylène glycol fonctionnalisé d'une masse moléculaire de 2 000 à 6 000, un acrylamide de N-alkyle ayant une longueur de chaîne en C₃ à C₆ et un cyanoacrylate d'alkyle ayant une longueur de chaîne en C₃ à C₆.

3. Formulation selon la revendication 1 ou 2, dans laquelle le copolymère est formé à partir de vinylpyrrolidone, d'acrylamide de N-isopropyle, et de polyéthylène glycol fonctionnalisé.

4. Formulation selon la revendication 1, 2 ou 3, dans laquelle la concentration du médicament est environ de 0,1 à 20 mg/mL de la formulation.

5. Formulation selon la revendication 1, 2, 3 ou 4, dans laquelle la concentration du copolymère est environ de 0,01 à 20 mg/mL de la formulation.

6. Formulation selon l'une quelconque des revendications 1 à 5, dans laquelle l'agent tensioactif anionique est le désoxycholate de sodium.

7. Formulation selon la revendication 6, dans laquelle la concentration du désoxycholate de sodium est environ de 0,01 à 20 mg/mL de la formulation.

8. Formulation selon la revendication 1, dans laquelle l'agent tampon est approprié pour une administration intraveineuse.

9. Formulation selon l'une quelconque des revendications 1 à 6, dans laquelle le pH est compris entre 6,0 et 7,5.

10. Formulation selon la revendication 1, dans laquelle le fluide de dilution aqueux intraveineux est une solution de dextrose.

11. Formulation selon l'une quelconque des revendications 1 à 6, dans laquelle la concentration du paclitaxel dans l'alcool est de 10 à 80 mg/mL.

12. Procédé de préparation d'une formulation selon la revendication 1, 2, 3, 4, 5, 6 ou 11 pour une administration intravasculaire, comprenant les étapes consistant :
a) à dissoudre un copolymère dans un fluide de dilution intraveineux ;
b) à ajouter un agent tensioactif anionique à la solution de l'étape « a » ;
c) à ajuster le pH de la solution résultante ; et
d) à ajouter une solution alcoolique de paclitaxel ou d'un dérivé de celui-ci à la solution ci-dessus.

13. Formulation préparée par le procédé de la revendication 12.

14. Formulation selon la revendication 1, 2, 3, 4, 5, 6 ou 11 pour le traitement d'un état émanant d'une prolifération excessive de cellules.

15. Formulation selon la revendication 14, dans laquelle l'état est un cancer.

16. Formulation selon la revendication 14, dans laquelle l'état est une tumeur.

17. Formulation selon la revendication 14, dans laquelle l'état est l'arthrite rhumatoïde.

18. Utilisation d'une formulation selon la revendication 1, 2, 3, 4, 5, 6 ou 11 pour la fabrication d'un médicament pour le traitement d'un état émanant de la prolifération excessive des cellules.
